(19) Europäisches Patentamt
European Patent Office
Office européen des brevets

(11) **EP 3 270 972 B1**

(12) **EUROPEAN PATENT SPECIFICATION**

(45) Date of publication and mention
of the grant of the patent:
**09.01.2019 Bulletin 2019/02**

(51) Int Cl.:
***A61K 47/38*** (2006.01)  ***A61K 9/16*** (2006.01)
***C08B 11/20*** (2006.01)  ***C08B 13/00*** (2006.01)

(21) Application number: **16711738.1**

(86) International application number:
**PCT/US2016/021334**

(22) Date of filing: **08.03.2016**

(87) International publication number:
**WO 2016/148978 (22.09.2016 Gazette 2016/38)**

(54) **PROCESS FOR PREPARING AN ESTERIFIED CELLULOSE ETHER IN THE PRESENCE OF AN ALIPHATIC CARBOXYLIC ACID**

VERFAHREN ZUR HERSTELLUNG EINES VERESTERTEN CELLULOSEETHERS IN GEGENWART EINER ALIPHATISCHEN CARBONSÄURE

MÉTHODE DE PRÉPARATION D'UN ÉTHER DE CELLULOSE ESTÉRIFIÉ EN PRÉSENCE D'UN ACIDE CARBOXILIQUE ALIPHATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.03.2015 US 201562133506 P**

(43) Date of publication of application:
**24.01.2018 Bulletin 2018/04**

(73) Proprietor: **Dow Global Technologies LLC Midland, MI 48674 (US)**

(72) Inventors:
• **PETERMANN, Oliver**
  **29699 Bomlitz (DE)**
• **APPELL, Robert B.**
  **Midland, MI 48674 (US)**

(74) Representative: **f & e patent**
  **Fleischer, Engels & Partner mbB, Patentanwälte**
  **Braunsberger Feld 29**
  **51429 Bergisch Gladbach (DE)**

(56) References cited:
**WO-A1-2014/031418    WO-A1-2014/031446
WO-A1-2014/031447    WO-A1-2014/137789**

**Description**

FIELD

The present invention relates to an improved process for preparing an ester of a cellulose ether in the presence of an aliphatic carboxylic acid.

INTRODUCTION

Esters of cellulose ethers, their uses and processes for preparing them are generally known in the art. U.S. Patent No. 4,226,981 and European Patent Application EP 0 219 426 disclose a process for preparing mixed esters of cellulose ethers, such as hydroxypropyl methyl cellulose acetate succinate (HPMCAS), by esterifying hydroxypropyl methylcellulose with succinic anhydride and acetic anhydride in the presence of an alkali metal carboxylate as the esterification catalyst and acetic acid as the reaction medium.

International Patent Applications WO2014/031447 and WO2014/031448 disclose the production of esters of cellulose ethers by esterifying cellulose ethers in the presence of an aliphatic carboxylic acid, such as acetic acid, and in the presence of an alkali metal carboxylate. WO2014/031447 and WO2014/031448, respectively, disclose that the weight average molecular weight of the produced esterified cellulose ethers depend on the molecular ratio of acetic acid / cellulose ether and alkali metal carboxylate / cellulose ether, respectively, that are utilized in the esterification process. International Patent Application WO2014/031446 discloses a process for producing esterified cellulose ethers which requires a reduced amount of alkali metal carboxylate and acetic acid.

It is also known in the prior art, e.g. as published in International Patent Application WO 2005/115330, that HPMCAS is useful to increase the bioavailability of poorly water-soluble drugs. This is of great importance as nearly 70% of new drug candidates are low water soluble compounds. As a general rule, poorly water soluble drugs possess low bioavailability. The HPMCAS is aimed at reducing the crystallinity of the drug, thereby minimizing the activation energy necessary for the dissolution of the drug, as well as establishing hydrophilic conditions around the drug molecules, thereby improving the solubility of the drug itself to increase its bioavailability, i.e., its *in vivo* absorption by an individual upon ingestion.

The solubility of HPMCAS in aqueous liquids is pH-dependent due to the presence of succinate groups, also called succinyl groups or succinoyl groups. HPMCAS is known as enteric polymer for pharmaceutical dosage forms. In the acidic environment of the stomach HPMCAS is protonated and therefore insoluble. HPMCAS undergoes deprotonation and becomes soluble in the small intestine, which is an environment of higher pH. The pH-dependent solubility is dependent on the degree of substitution of acidic functional groups. The dissolution time of various types of HPMCAS dependent on pH and on the degree of neutralization of HPMCAS is discussed in detail in McGinity, James W. Aqueous Polymeric Coatings for Pharmaceutical Dosage Forms, New York: M. Dekker, 1989, pages 105 - 113. The above-mentioned article *Aqueous Polymeric Coatings for Pharmaceutical Dosage Forms* illustrates in Fig. 16 on p. 112 the dissolution time of several grades of HPMCAS, which have different degrees of substitution with succinoyl, acetyl and methoxyl groups, in pure water and in 0.1 N NaCl depending on the degree of neutralization of the HPMCAS. Depending on the HPMCAS and the presence or absence of NaCl, HPMCAS is soluble when it has a degree of neutralization between about 0.55 and 1. Below a degree of neutralization of about 0.55, all HPMCAS grades are insoluble in pure water and in 0.1 N NaCl.

Dosage forms coated with esterified cellulose ethers such as HPMCAS protect the drug from inactivation or degradation in the acidic environment of the stomach or prevent irritation of the stomach by the drug but release the drug in the small intestine. US Patent No. 4,365,060 discloses enterosoluble capsules. U.S. Patent No. 4,226,981 discloses a process for preparing mixed esters of cellulose ethers, such as HPMCAS.

US Patent No. 4,365,060 discloses enterosoluble capsules which are said to have excellent enterosolubility behavior. The capsules are prepared from a mixed ester of an alkyl-, hydroxyalkyl- or hydroxyalkyl-alkyl- cellulose esterified with succinyl anhydride and an aliphatic monocarboxylic acid anhydride. The US patent discloses that the cellulose derivative can be shaped into capsules not only by the conventional dipping method but also by the plastic deformation at an elevated temperature under pressure such as compression molding, vacuum forming, matched-mold forming and the like. The US patent states that the enterosoluble capsules have excellent pliability. However, forming capsules by plastic deformation is often not desirable due to the significant thermal stress and thermal degradation caused by the heat that is needed for thermoforming or the complex and expensive molding process for thermoforming thin film capsules. On the other hand, in the known dipping method molding pins are dipped into a solution of the mixed ester of an alkyl-, hydroxyalkyl- or hydroxyalkyl-alkyl- cellulose in an organic solvent. Organic solutions of alkyl-, hydroxyalkyl- or hydroxyalkyl-alkyl- celluloses can also be used for coating dosage forms, such as tablets. However, organic solvents are often not desirable for pharmaceutical or nutritional uses. Moreover, the handling of organic solvents adds to the complexity of the process for producing capsules and coatings.

In view of the great importance of esterified cellulose ethers such as HPMCAS for increasing the bioavailability of poorly water-soluble drugs, there is an urgent need to find an improved, less expensive process for producing esterified cellulose ethers. It would be particularly desirable if an improved, less expensive process for producing esterified cellulose ethers could be found, which allows in at least some embodiments of the process the production of water-soluble esterified cellulose ethers.

SUMMARY

Surprisingly, it has been found that esterified cellulose ethers can be produced in the presence of an aliphatic carboxylic acid but in the substantial or even complete absence of an esterification catalyst, such as alkali metal carboxylate.

Accordingly, one aspect of the present invention is a process for preparing an ester of a cellulose ether which comprises the steps of reacting a cellulose ether with a dicarboxylic acid anhydride or with a combination of a dicarboxylic acid anhydride and an aliphatic monocarboxylic acid anhydride, wherein the esterification is conducted

   i) in the absence of an esterification catalyst or in the presence of no more than 0.1 mole of an esterification catalyst per mole of anhydroglucose units of cellulose ether, and
   ii) in the presence of an aliphatic carboxylic acid, wherein the molar ratio [aliphatic carboxylic acid / anhydroglucose units of cellulose ether] is up to 12/1.

The novel process results in substantial savings in raw materials and savings in the subsequent purification processes. The large amount of alkali metal carboxylate, which is used in known esterification processes and which needs to be recovered after the esterification reaction, is not needed on the process of the present invention.

Even more surprisingly, it has been found that new, water-soluble esterified cellulose ethers can be produced by the above-mentioned process. Hence, another aspect of the present invention is an esterified cellulose ether which is producible by the above-mentioned process.

BRIEF DESCRIPTION OF DRAWINGS

   Fig. 1 is a photographical representation of solutions of esterified cellulose ethers in water which have been produced by Examples 7 - 11 of the present invention.
   Fig. 2A, 3A and 4A are photographical representations of capsule shells on metallic pins having a temperature of 21 °C, 30 °C and 55 °C, respectively.
   Fig. 2B, 3B and 4B are photographical representations of pieces of capsule shells formed on metallic pins having a temperature of 21 °C, 30 °C and 55 °C, respectively, after the capsule shells have been removed from the dipping pins.
   Fig. 2C, 3C and 4C are photographical representations of non-dissolved pieces of capsule shells in 0.1 N HCl. The pieces of capsule shells are small pieces of the capsules shells represented in Fig. 2B, 3B and 4B, respectively.
   Fig. 2D, 3D and 4D are photographical representations of an aqueous buffer solution of pH 6.8 into which the non-dissolved pieces of capsule shells shown in Fig. 2C, 3C and 4C have been placed; all pieces of capsule shells are dissolved in the aqueous buffer solution of pH 6.8.

DESCRIPTION OF EMBODIMENTS

The cellulose ether used as a starting material in the process of the present invention has a cellulose backbone having $\beta$-1,4 glycosidically bound D-glucopyranose repeating units, designated as anhydroglucose units in the context of this invention. The cellulose ether preferably is an alkyl cellulose, hydroxyalkyl cellulose or hydroxyalkyl alkylcellulose. This means that in the cellulose ether utilized in the process of the present invention, at least a part of the hydroxyl groups of the anhydroglucose units are substituted by alkoxyl groups or hydroxyalkoxyl groups or a combination of alkoxyl and hydroxyalkoxyl groups. The hydroxyalkoxyl groups are typically hydroxymethoxyl, hydroxyethoxyl and/or hydroxypropoxyl groups. Hydroxyethoxyl and/or hydroxypropoxyl groups are preferred. Typically one or two kinds of hydroxyalkoxyl groups are present in the cellulose ether. Preferably a single kind of hydroxyalkoxyl group, more preferably hydroxypropoxyl, is present. The alkoxyl groups are typically methoxyl, ethoxyl and/or propoxyl groups. Methoxyl groups are preferred.

Illustrative of the above-defined cellulose ethers are alkylcelluloses, such as methylcellulose, ethylcellulose, and propylcellulose; hydroxyalkylcelluloses, such as hydroxyethylcellulose, hydroxypropylcellulose, and hydroxybutylcellulose; and hydroxyalkyl alkylcelluloses, such as hydroxyethyl methylcellulose, hydroxymethyl ethylcellulose, ethyl hydroxyethylcellulose, hydroxypropyl methylcellulose, hydroxypropyl ethylcellulose, hydroxybutyl methylcellulose, and hydroxybutyl ethylcellulose; and those having two or more hydroxyalkyl groups, such as hydroxyethylhydroxypropyl methylcellulose. Most preferably, the cellulose ether is a hydroxyalkyl methylcellulose, such as hydroxypropyl methylcellulose.

The degree of the substitution of hydroxyl groups of the anhydroglucose units by hydroxyalkoxyl groups is expressed by the molar substitution of hydroxyalkoxyl groups, the MS(hydroxyalkoxyl). The MS(hydroxyalkoxyl) is the average number of moles of hydroxyalkoxyl groups per anhydroglucose unit in the cellulose ether. It is to be understood that during the hydroxyalkylation reaction the hydroxyl group of a hydroxyalkoxyl group bound to the cellulose backbone can be further etherified by an alkylation agent, e.g. a methylation agent, and/or a hydroxyalkylation agent. Multiple subsequent hydroxyalkylation etherification reactions with respect to the same carbon atom position of an anhydroglucose unit yields a side chain, wherein multiple hydroxyalkoxyl groups are covalently bound to each other by ether bonds, each side chain as a whole forming a hydroxyalkoxyl substituent to the cellulose backbone.

The term "hydroxyalkoxyl groups" thus has to be interpreted in the context of the MS(hydroxyalkoxyl) as referring to the hydroxyalkoxyl groups as the constituting units of hydroxyalkoxyl substituents, which either comprise a single hydroxyalkoxyl group or a side chain as outlined above, wherein two or more hydroxyalkoxy units are covalently bound to each other by ether bonding. Within this definition it is not important whether the terminal hydroxyl group of a hydroxyalkoxyl substituent is further alkylated, e.g. methylated, or not; both alkylated and non-alkylated hydroxyalkoxyl substituents are included for the determination of MS(hydroxyalkoxyl). The cellulose ether utilized in the process of the invention generally has a molar substitution of hydroxyalkoxyl groups in the range 0.05 to 1.00, preferably 0.08 to 0.90, more preferably 0.12 to 0.70, most preferably 0.15 to 0.60, and particularly 0.20 to 0.40.

The average number of hydroxyl groups substituted by alkoxyl groups, such as methoxyl groups, per anhydroglucose unit, is designated as the degree of substitution of alkoxyl groups, DS(alkoxyl). In the above-given definition of DS, the term "hydroxyl groups substituted by alkoxyl groups" is to be construed within the present invention to include not only alkylated hydroxyl groups directly bound to the carbon atoms of the cellulose backbone, but also alkylated hydroxyl groups of hydroxyalkoxyl substituents bound to the cellulose backbone. The cellulose ethers according to this invention generally have a DS(alkoxyl) in the range of 1.0 to 2.5, preferably from 1.1 to 2.4, more preferably from 1.2 to 2.2 most preferably from 1.6 to 2.05, and particularly from 1.7 to 2.05.

The degree of substitution of alkoxyl groups and the molar substitution of hydroxyalkoxyl groups can be determined by Zeisel cleavage of the cellulose ether with hydrogen iodide and subsequent quantitative gas chromatographic analysis (G. Bartelmus and R. Ketterer, Z. Anal. Chem., 286 (1977) 161-190). Most preferably the cellulose ether utilized in the process of the invention is hydroxypropyl methylcellulose having a DS(methoxyl) within the ranges indicated above for DS(alkoxyl) and an MS(hydroxypropoxyl) within the ranges indicated above for MS(hydroxyalkoxyl).

The cellulose ether used as a starting material in the process of the present invention generally has a viscosity of up to 200 mPa·s, preferably up to 100 mPa·s, more preferably up to 50 mPa·s, and most preferably up to 5 mPa·s, measured as a 2 weight-% aqueous solution at 20 °C according to ASTM D2363 - 79 (Reapproved 2006). Generally their viscosity is at least 1.2 mPa·s, typically at least 1.8 mPa·s, even more typically at least 2.4 mPa·s, and most typically at least 2.8 mPa·s, measured as a 2 weight-% aqueous solution at 20 °C. Cellulose ethers of such viscosity can be obtained by subjecting a cellulose ether of higher viscosity to a partial depolymerization process. Partial depolymerization processes are well known in the art and described, for example, in European Patent Applications EP 1,141,029; EP 210,917; EP 1,423,433; and US Patent No. 4,316,982. Alternatively, partial depolymerization can be achieved during the production of the cellulose ethers, for example by the presence of oxygen or an oxidizing agent.

The cellulose ether is reacted with (i) a dicarboxylic acid anhydride or (ii), more preferably, with a combination of an aliphatic monocarboxylic acid anhydride and a dicarboxylic acid anhydride. Preferred aliphatic monocarboxylic acid anhydrides are selected from the group consisting of acetic anhydride, butyric anhydride and propionic anhydride. A preferred dicarboxylic acid anhydride is succinic anhydride. A preferred dicarboxylic acid anhydride can be used alone or a preferred aliphatic monocarboxylic acid anhydride can be used in combination with a preferred dicarboxylic acid anhydride. More preferably the cellulose ether is esterified with succinic anhydride in combination with an aliphatic monocarboxylic acid anhydride selected from the group consisting of acetic anhydride, butyric anhydride and propionic anhydride. If an aliphatic monocarboxylic acid anhydride and a dicarboxylic acid anhydride are used for esterifying the cellulose ether, the two anhydrides may be introduced into the reaction vessel at the same time or separately one after the other. Most preferably, hydroxypropyl methylcellulose is reacted with succinic anhydride and acetic anhydride to produce hydroxypropyl methyl cellulose acetate succinate.

The molar ratio between the anhydride of an aliphatic monocarboxylic acid and the anhydroglucose units of the cellulose ether generally is 0.1 / 1 or more, preferably 0.3 / 1 or more, and more preferably 0.5 / 1 or more. The molar ratio between the anhydride of an aliphatic monocarboxylic acid and the anhydroglucose units of the cellulose ether generally is 17 / 1 or less, preferably 10 / 1 or less, more preferably 7 / 1 or less, most preferably 4/1 or less, and particularly 3.5 / 1 or less.

The molar ratio between the anhydride of a dicarboxylic acid and the anhydroglucose units of cellulose ether generally is 0.01/1 or more, preferably 0.05 / 1 or more, more preferably 0.1 / 1 or more, most preferably 0.2/1 or more, and particularly preferably 0.3 / 1 or more. The molar ratio between the anhydride of a dicarboxylic acid and the anhydroglucose units of cellulose ether generally is 3.2/1 or less, preferably 2.5 / 1 or less, more preferably 1.5/1 or less, most preferably 1.2/1 or less, and particularly 0.7 / 1 or less.

The molar number of anhydroglucose units of the cellulose ether utilized in the process of the present invention can be determined from the weight of the cellulose ether used as a starting material, by calculating the average molecular weight of the substituted anhydroglucose units from the DS(alkoxyl) and MS(hydroxyalkoxyl).

In known esterification processes the cellulose ether is reacted with an aliphatic monocarboxylic acid anhydride and/or a dicarboxylic acid anhydride in the presence of a large amount of an esterification catalyst, typically an alkali metal carboxylate, such as sodium acetate or potassium acetate. In contrast to known processes, the process of the present invention is conducted in the absence of an esterification catalyst or in the presence of no more than 0.1 mole, preferably no more than 0.05 mole, more preferably no more than 0.02 mole, and most preferably no more than 0.01 mole of an esterification catalyst, such as an alkali metal carboxylate, per mole of anhydroglucose units of cellulose ether. Most preferably, the process of the present invention is conducted in the absence of an esterification catalyst. It is surprising and contrary to the teaching of the prior art that cellulose ethers can be esterified in the presence of an aliphatic carboxylic acid but in the substantial or even complete absence of an esterification catalyst, such as an alkali metal carboxylate. The novel process results in substantial savings in raw materials and savings in the subsequent purification processes. The large amount of alkali metal carboxylate, which is used in known esterification processes and which needs to be recovered after the esterification reaction, is not needed in the process of the present invention. Moreover, improved use of existing reactor capacity can be made because higher loading of the reactor with cellulose ether, dicarboxylic acid anhydride and aliphatic monocarboxylic acid anhydride is possible by carrying out the esterification reaction in the substantial or even complete absence of an esterification catalyst. Even more surprisingly, it has been found that in at least some embodiments of the present invention water-soluble esterified cellulose ethers can be produced, as evidenced by the Examples.

The esterification of the cellulose ether is conducted in an aliphatic carboxylic acid as a reaction diluent, such as acetic acid, propionic acid, or butyric acid. The reaction diluent can comprise minor amounts of other solvents or diluents which are liquid at room temperature and do not react with the cellulose ether, such as aromatic or aliphatic solvents like benzene, toluene, 1,4-dioxane, or tetrahydrofurane; or halogenated $C_1$-$C_3$ derivatives, like dichloro methane or dichloro methyl ether, but the amount of the aliphatic carboxylic acid should generally be more than 50 percent, preferably at least 75 percent, and more preferably at least 90 percent, based on the total weight of the reaction diluent. Most preferably the reaction diluent consists of an aliphatic carboxylic acid. The molar ratio [aliphatic carboxylic acid / anhydroglucose units of cellulose ether] is up to 12/1, generally up to 10 / 1, and preferably up to 9.0 / 1. In some embodiments of the invention lower ratios, such as up to 7.0 / 1, even only up to 4.0 / 1 or even up to 2.0 / 1 can also be used, which makes optimal use of the amount of the aliphatic carboxylic acid needed as reaction diluent. The molar ratio [aliphatic carboxylic acid / anhydroglucose units of cellulose ether] generally is at least 0.7 / 1, preferably at least 1.2 / 1, more preferably at least 1.5 / 1, and most preferably at least 3.0 / 1. When the molar ratio [aliphatic carboxylic acid / anhydroglucose units of cellulose ether] is from [2.0 / 1] to [9.0 / 1] and in particular from [3.0 / 1] to [9.0 / 1], esterified cellulose ethers can be obtained that form very clear solutions in water, as described further below. The reaction temperature for the esterification is generally from 60° C to 110 ° C, preferably from 70 ° C to 100 ° C. The esterification reaction is typically completed within 2 to 25 hours, more typically within 2 to 8 hours. After completion of the esterification reaction, the reaction product can be precipitated from the reaction mixture in a known manner, for example by contacting the reaction mixture with a large volume of water, such as described in U.S. Patent No. 4,226,981, International Patent Application WO 2005/115330 or European Patent Application EP 0 219 426. In a preferred embodiment of the invention the reaction product is precipitated from the reaction mixture as described in International Patent Application PCT/US13/030394, published as WO2013/148154, to produce an esterified cellulose ether in the form of a powder.

By the process of the present invention esterified cellulose ethers are produced which comprise groups of the formula -C(O)-R-COOH, wherein R is a divalent hydrocarbon group, such as -C(O)-$CH_2$-$CH_2$-COOH, and optionally aliphatic monovalent acyl groups, such as acetyl, propionyl, or butyryl, such as n-butyryl or i-butyryl. Specific examples of esterified cellulose ethers are hydroxypropyl methylcellulose acetate succinate (HPMCAS), hydroxypropyl cellulose acetate succinate (HPCAS), hydroxybutyl methyl cellulose propionate succinate (HBMCPrS), hydroxyethyl hydroxypropyl cellulose propionate succinate (HEHPCPrS), or methyl cellulose acetate succinate (MCAS). Hydroxypropyl methylcellulose acetate succinate (HPMCAS) is the most preferred esterified cellulose ether.

The esterified cellulose ethers produced according to the process of the present invention generally have a degree of substitution of aliphatic monovalent acyl groups, such as acetyl, propionyl, or butyryl groups, of 0 or at least 0.05, preferably at least 0.10, and more preferably at least 0.25. The esterified cellulose ethers generally have a degree of substitution of aliphatic monovalent acyl groups of up to 1.5, preferably up to 1.0, and more preferably up to 0.6. The esterified cellulose ethers of the present invention generally have a degree of substitution of groups of formula -C(O)-R-COOH, such as succinoyl, of at least 0.01, preferably at least 0.05, and most preferably at least 0.10. The esterified cellulose ethers generally have a degree of substitution of groups of formula -C(O)-R-COOH of up to 1.3, preferably up to 0.8, and more preferably up to 0.5.

The total degree of ester substitution is generally at least 0.05, preferably at least 0.10, more preferably at least 0.20, and most preferably at least 0.25. The total degree of ester substitution is generally not more than 1.5, preferably not more than 1.2, more preferably not more than 0.90 and most preferably not more than 0.70.

The content of the acetate and succinate ester groups is determined according to "Hypromellose Acetate Succinate, United States Pharmacopia and National Formulary, NF 29, pp. 1548-1550". Reported values are corrected for volatiles (determined as described in section "loss on drying" in the above HPMCAS monograph). The method may be used in analogue manner to determine the content of propionyl, butyryl and other ester groups. The content of ether groups in the esterified cellulose ether is determined in the same manner as described for "Hypromellose", United States Pharmacopeia and National Formulary, USP 35, pp 3467-3469. The contents of ether and ester groups obtained by the above analyses are converted to DS and MS values of individual substituents according to the formulas below. The formulas may be used in analogue manner to determine the DS and MS of substituents of other cellulose ether esters.

$$
\begin{aligned}
\% \text{ cellulose backbone} \\
= 100 - \left( \%\text{MeO} * \frac{M(OCH_3) - M(OH)}{M(OCH_3)} \right) \\
- \left( \%\text{HPO} * \frac{M(OCH_2CH(OH)CH_3) - M(OH)}{M(OCH_2CH(OH)CH_3)} \right) \\
- \left( \%\text{Acetyl} * \frac{M(COCH_3) - M(H)}{M(COCH_3)} \right) \\
- \left( \%\text{Succinoyl} * \frac{M(COC_2H_4COOH) - M(H)}{M(COC_2H_4COOH)} \right)
\end{aligned}
$$

$$
DS(Me) = \frac{\frac{\%\text{MeO}}{M(OCH_3)}}{\frac{\%\text{cellulose backbone}}{M(AGU)}}
$$

$$
MS(HP) = \frac{\frac{\%\text{HPO}}{M(HPO)}}{\frac{\%\text{cellulose backbone}}{M(AGU)}}
$$

$$
DS(Acetyl) = \frac{\frac{\%\text{Acetyl}}{M(Acetyl)}}{\frac{\%\text{cellulose backbone}}{M(AGU)}}
$$

$$
DS(Succinoyl) = \frac{\frac{\%\text{Succinoyl}}{M(Succinoyl)}}{\frac{\%\text{cellulose backbone}}{M(AGU)}}
$$

M(MeO) = M(OCH_3) = 31.03 Da M(HPO) = M(OCH_2CH(OH)CH_3) = 75.09 Da M(Acetyl) = M(COCH_3) = 43.04 Da M(Succinoyl) = M(COC_2H_4COOH) = 101.08 Da M(AGU) = 162.14 Da M(OH) = 17.008 Da M(H) = 1.008 Da

By convention, the weight percent is an average weight percentage based on the total weight of the cellulose repeat unit, including all substituents. The content of the methoxyl group is reported based on the mass of the methoxyl group (i.e., -OCH_3). The content of the hydroxyalkoxyl group is reported based on the mass of the hydroxyalkoxyl group (i.e., -O-alkylene-OH); such as hydroxypropoxyl (i.e., -O-CH_2CH(CH_3)-OH). The content of the aliphatic monovalent acyl groups is reported based on the mass of $-C(O)-R_1$ wherein $R_1$ is a monovalent aliphatic group, such as acetyl ($-C(O)-CH_3$). The content of the group of formula $-C(O)-R-COOH$ is reported based on the mass of this group, such as the mass of succinoyl groups (i.e., $- C(O)-CH_2-CH_2-COOH$).

The esterified cellulose ethers which are produced by the process of the present invention generally have a weight average molecular weight $M_w$ of up to 500,000 Dalton, preferably up to 250,000 Dalton, more preferably up to 200,000 Dalton, most preferably up to 150,000 Dalton, and particularly up to 100,000 Dalton.

Generally they have a weight average molecular weight $M_w$ of at least 10,000 Dalton, preferably at least 12,000 Dalton, more preferably at least 15,000 Dalton, most preferably at least 20,000 Dalton, and particularly at least 30,000 Dalton.

The esterified cellulose ethers of the present invention generally have a Polydispersity $M_w/M_n$, i.e., a ratio of weight average molecular weight $M_w$ to number average molecular weight $M_n$, of at least 1.2, typically at least 1.3 and often at least 1.5. Moreover, the esterified cellulose ethers of the present invention generally have a Polydispersity of up to 4.1, preferably of up to 3.9, and most preferably of up to 3.7.

$M_w$ and $M_n$ are measured according to Journal of Pharmaceutical and Biomedical Analysis 56 (2011) 743 using a mixture of 40 parts by volume of acetonitrile and 60 parts by volume of aqueous buffer containing 50 mM $NaH_2PO_4$ and 0.1 M $NaNO_3$ as mobile phase. The mobile phase is adjusted to a pH of 8.0. The measurement of $M_w$ and $M_n$ is described in more details in the Examples.

Surprisingly, in at least some embodiments of the process of the present invention esterified cellulose ethers are produced which can be dissolved as at least 2.0 weight percent solutions, preferably at least 3.0 weight percent solutions, and more preferably at least 5.0 weight percent solutions in water at 2 °C. Generally these esterified cellulose ethers can be dissolved as up to 20 weight percent solutions or in the most preferred embodiments even as up to 30 weight percent solutions in water at a temperature of 2 °C. The term "an x weight percent solution in water at 2 °C" as used herein means that x g of the esterified cellulose ether is soluble in (100 - x) g of water at 2 °C.

When determining the water solubility as described in the Examples section, in at least some embodiments of the process of the present invention esterified cellulose ethers are produced which have solubility properties that at least 80 wt.%, typically at least 85 wt.%, more typically at least 90 wt.%, and in most cases at least 95 wt.% of the esterified cellulose ether is soluble in a mixture of 2.5 weight parts of the esterified cellulose ether and 97.5 weight parts of water at 2 °C. Typically this degree of solubility is also observed in a mixture of 5 or 10 weight parts of the esterified cellulose ether and 95 or 90 weight parts of water at 2 °C or even in a mixture of 20 weight parts of the esterified cellulose ether and 80 weight parts of water at 2 °C.

The esterified cellulose ethers have these surprising solubilities even in their predominantly or substantially protonated form, i.e., when the degree of neutralization of the groups $- C(O) - R - COOH$ is not more than 0.4, preferably not more than 0.3, more preferably not more than 0.2, most preferably not more than 0.1, and particularly not more than 0.05 or even not more than 0.01. The degree of neutralization can even be essentially zero or only slightly above it, e.g. up to $10^{-3}$ or even only up to $10^{-4}$. The term "degree of neutralization" as used herein defines the ratio of deprotonated carboxylic groups over the sum of deprotonated and protonated carboxylic groups, i.e.,

$$\text{Degree of neutralization} = [-C(O)-R-COO^-] \,/\, [-C(O)-R-COO^- + -C(O)-R-COOH].$$

Some embodiments of the invention will now be described in detail in the following Examples.

EXAMPLES

Unless otherwise mentioned, all parts and percentages are by weight. In the Examples the following test procedures are used.

Content of ether and ester groups

The content of ether groups in the esterified cellulose ether is determined in the same manner as described for "Hypromellose", United States Pharmacopeia and National Formulary, USP 35, pp 3467-3469.

The ester substitution with acetyl groups ($-CO-CH_3$) and the ester substitution with succinyl groups ($-CO-CH_2-CH_2-COOH$) are determined according to Hypromellose Acetate Succinate, United States Pharmacopia and National Formulary, NF 29, pp. 1548-1550". Reported values for ester substitution are corrected for volatiles (determined as described in section "loss on drying" in the above HPMCAS monograph).

Determination of $M_w$ and $M_n$

$M_w$ and $M_n$ are measured according to Journal of Pharmaceutical and Biomedical Analysis 56 (2011) 743 unless stated otherwise. The mobile phase was a mixture of 40 parts by volume of acetonitrile and 60 parts by volume of aqueous buffer containing 50 mM $NaH_2PO_4$ and 0.1 M $NaNO_3$. The mobile phase was adjusted to a pH of 8.0. Solutions of the cellulose ether esters were filtered into a HPLC vial through a syringe filter of 0.45 μm pore size. The exact details of measuring $M_w$ and $M_n$ are disclosed in the International Patent Application No. WO 2014/137777 in the section "Examples" under the title "Determination of $M_w$, $M_n$ and $M_z$". In all Examples of the invention the recovery rate was at least 96 %. In the Comparative Examples the recovery rate was at least 89 %.

Water-Solubility

Qualitative determination: A 2 wt. percent mixture of HPMCAS and water was prepared by mixing 2.0 g HPMCAS, based on its dry weight, with 98.0 g water under vigorous stirring at 0.5°C for 16 hours. The temperature of the mixture of HPMCAS and water was then increased to 5 °C. The water solubility of the esterified cellulose ether was determined by visual inspection. The determination whether the HPMCAS was water-soluble at 2% at 5 °C or not was done as follows. "Water soluble at 2% - yes" means that a solution without sediment was obtained according to the procedure above. "Water soluble at 2% - no" means that at least a significant portion of the HPMCAS remained undissolved and formed sediment when mixing 2.0 g HPMCAS, based on its dry weight, with 98.0 g water according to the procedure above. "Water soluble at 2% - partially" means that only a small portion of the HPMCAS remained undissolved and formed sediment when mixing 2.0 g HPMCAS, based on its dry weight, with 98.0 g water according to the procedure above.

Quantitative determination: 2.5 weight parts of HPMCAS, based on its dry weight, were added to 97.5 weight parts of deionized water having a temperature of 2 °C followed by stirring for 6 hours at 2°C and storing for 16 h at 2°C. A weighed amount of this mixture was transferred to a weighed centrifuge vial; the transferred weight of the mixture was noted as M1 in g. The transferred weight of HPMCAS [M2] was calculated as (transferred weight of mixture in g/100 g * 2.5g). The mixture was centrifuged for 60 min at 5000 rpm (2823 xg, Biofuge Stratos centrifuge from Thermo Scientific) at 2 °C. After centrifugation an aliquot was removed from the liquid phase and transferred to a dried weighed vial. The weight of the transferred aliquot was recorded as M3 in g. The aliquot was dried at 105°C for 12 h. The remaining g of HPMCAS was weighted after drying and recorded as M4 in g.

The term "% water soluble at 2.5 %" in Table 2 below expresses the percentage of HPMCAS that is actually dissolved in the mixture of 2.5 weight parts of HPMCAS and 97.5 weight parts of deionized water. It is calculated as (M4 / M2) * (M1 / M3) * 100), which corresponds to (g HPMCAS in liquid aliquot / g HPMCAS transferred to centrifuge vial) * (g mixture transferred to centrifuge vial / g liquid aliquot after centrifugation).

Viscosity of Hydroxypropyl Methyl Cellulose Acetate Succinate (HPMCAS)

The 2.0 % by weight solution of the HPMCAS in 0.43 wt. % aqueous NaOH was prepared as described in"Hypromellose Acetate Succinate, United States Pharmacopia and National Formulary, NF 29, pp. 1548-1550". An Ubbelohde viscosity measurement according to DIN 51562-1:1999-01 (January 1999) was carried out. The measurement was done at 20 °C. The 2.0 % by weight solution of the HPMCAS in 0.43 wt.% aqueous NaOH is listed in Table 2 below as "2.0 % viscosity in NaOH for those Examples and Comparative Examples for which this property had been measured.

The 10 wt.-% solution of HPMCAS in acetone was prepared by mixing 10.0 g HPMCAS, based on its dry weight, with 90.0 g of acetone under vigorous stirring at room temperature. The mixture was rolled on a roller mixer for about 24 hours. The solution was centrifuged at 2000 rpm for 3 minutes using a Megafuge 1.0 centrifuge, commercially available from Heraeus Holding GmbH, Germany. An Ubbelohde viscosity measurement according to DIN 51562-1:1999-01 (January 1999) was carried out. The measurement was done at 20 °C.

Production of HPMCAS of Examples 1- 27

Succinic anhydride and acetic anhydride was dissolved at 70°C in glacial acetic acid. Then hydroxypropyl methyl cellulose (HPMC, water free) was added under stirring. The amounts are listed in Table 1 below. The amount of HPMC is calculated on a dried basis. No amount of sodium acetate was added.

The HPMC had a methoxyl substitution ($DS_M$) and hydroxypropoxyl substitution ($MS_{HP}$) as listed in Table 2 below and a viscosity of 3.0 mPa·s, measured as a 2 % solution in water at 20 °C according to ASTM D2363 - 79 (Reapproved 2006). The weight average molecular weight of the HPMC was about 20,000 Dalton. The HPMC is commercially available from The Dow Chemical Company as Methocel E3 LV Premium cellulose ether.

Then the reaction mixture was heated up to the reaction temperature listed in Table 1 below. The reaction time during which the mixture was allowed to react is also listed in Table 1 below. Then the crude product was precipitated by adding 1 - 2 L of water having a temperature of 21 °C. Subsequently the precipitated product was separated from the mixture by filtration and washed several times with water having the temperature listed in Table 1 below. Then the product was isolated by filtration and dried at 55°C overnight.

For Example 23 the precipitated reaction mass was split in two halves. The first halve was washed with water having a temperature of 21 °C (Example 23). The second halve was washed with water having a temperature of 95°C (Example 23A).

Production of HPMCAS of Comparative Examples A - E

Comparative Examples A - E were produced as described for Examples 1 - 27, except that sodium acetate was mixed with the other reactants in the amounts listed in Table 1 below. Comparative Examples A - E are for comparative purposes, but have not been described in the prior art.

The properties of the HPMCAS of Examples 1 - 27 and Comparative Examples A - E are listed in Table 2 below. In Table 2 the abbreviations have the following meanings:

$DS_M$ = DS(methoxyl): degree of substitution with methoxyl groups;
$MS_{HP}$ = MS(hydroxypropoxyl): molar subst. with hydroxypropoxyl groups;
$DS_{Ac}$: degree of substitution of acetyl groups;
$DS_s$: degree of substitution of succinoyl groups.

Table 1

| (Comp.) Example | HPMC* g | HPMC* Mol | Glacial acetic acid g | Glacial acetic acid mol/mol HPMC | Succinic anhydride g | Succinic anhydride mol/mol HPMC | Acetic anhydride g | Acetic anhydride mol/mol HPMC | Sodium acetate g | Sodium acetate mol/mol HPMC | Reaction temperature (°C) | Reaction time (hours) | Temperature of washing water, °C |
|---|---|---|---|---|---|---|---|---|---|---|---|---|---|
| 1 | 195 | 0.96 | 440 | 7.6 | 35 | 0.36 | 180 | 1.91 | 0 | 0 | 85 | 3 | 95 |
| 2 | 195 | 0.96 | 400 | 6.9 | 35 | 0.36 | 180 | 1.91 | 0 | 0 | 85 | 3 | 95 |
| 3 | 195 | 0.96 | 300 | 5.2 | 35 | 0.36 | 180 | 1.91 | 0 | 0 | 85 | 3 | 95 |
| 4 | 195 | 0.96 | 200 | 3.5 | 35 | 0.36 | 180 | 1.91 | 0 | 0 | 85 | 3 | 95 |
| 5 | 195 | 0.96 | 100 | 1.7 | 35 | 0.36 | 180 | 1.91 | 0 | 0 | 85 | 3 | 95 |
| 6 | 195 | 0.96 | 500 | 8.7 | 50 | 0.52 | 150 | 1.59 | 0 | 0 | 85 | 3 | 95 |
| 7 | 195 | 0.96 | 400 | 6.9 | 50 | 0.52 | 100 | 1.06 | 0 | 0 | 85 | 3 | 95 |
| 8 | 195 | 0.96 | 400 | 6.9 | 70 | 0.73 | 80 | 0.85 | 0 | 0 | 85 | 3 | 95 |
| 9 | 195 | 0.96 | 400 | 6.9 | 70 | 0.73 | 50 | 0.53 | 0 | 0 | 85 | 3 | 95 |
| 10 | 195 | 0.96 | 400 | 6.9 | 50 | 0.52 | 100 | 1.06 | 0 | 0 | 85 | 4.5 | 95 |
| 11 | 195 | 0.96 | 100 | 1.7 | 50 | 0.52 | 150 | 1.59 | 0 | 0 | 85 | 3 | 95 |
| 12 | 195 | 0.96 | 100 | 1.7 | 50 | 0.52 | 100 | 1.06 | 0 | 0 | 85 | 3 | 95 |
| 13 | 195 | 0.96 | 100 | 1.7 | 70 | 0.73 | 70 | 0.74 | 0 | 0 | 85 | 3 | 95 |
| 14 | 195 | 0.96 | 100 | 1.7 | 50 | 0.52 | 150 | 1.59 | 0 | 0 | 85 | 4.5 | 95 |
| 15 | 195 | 0.96 | 100 | 1.7 | 50 | 0.52 | 150 | 1.59 | 0 | 0 | 90 | 4 | 95 |
| 16 | 195 | 0.96 | 100 | 1.7 | 50 | 0.52 | 150 | 1.59 | 0 | 0 | 90 | 5 | 95 |
| 17 | 195 | 0.96 | 100 | 1.7 | 50 | 0.52 | 150 | 1.59 | 0 | 0 | 90 | 6 | 95 |
| 18 | 195 | 0.96 | 100 | 1.7 | 100 | 1.04 | 300 | 3.18 | 0 | 0 | 90 | 3 | 95 |
| 19 | 195 | 0.96 | 100 | 1.7 | 100 | 1.04 | 300 | 3.18 | 0 | 0 | 90 | 6 | 21 |
| 20 | 195 | 0.96 | 50 | 0.9 | 50 | 0.52 | 150 | 1.59 | 0 | 0 | 90 | 6 | 21 |
| 21 | 195 | 0.96 | 150 | 2.6 | 50 | 0.52 | 150 | 1.59 | 0 | 0 | 90 | 6 | 21 |
| 22 | 195 | 0.96 | 200 | 3.5 | 50 | 0.52 | 150 | 1.59 | 0 | 0 | 90 | 6 | 21 |
| 23 | 195 | 0.96 | 250 | 4.4 | 50 | 0.52 | 150 | 1.59 | 0 | 0 | 90 | 6 | 21 |

(continued)

| Table 1 | HPMC* | | Glacial acetic acid | | Succinic anhydride | | Acetic anhydride | | Sodium acetate | | Reaction temperature (°C) | Reaction time (hours) | Temperature of washing water, °C |
|---|---|---|---|---|---|---|---|---|---|---|---|---|---|
| (Comp.) Example | g | Mol | g | mol/mol HPMC | g | mol/mol HPMC | g | mol/mol HPMC | g | mol/mol HPMC | | | |
| 23A | 195 | 0.96 | 250 | 4.4 | 50 | 0.52 | 150 | 1.59 | 0 | 0 | 90 | 6 | 95 |
| A | 195 | 0.96 | 300 | 5.2 | 50 | 0.52 | 150 | 1.59 | 100 | 1.27 | 90 | 4 | 21 |
| B | 195 | 0.96 | 300 | 5.2 | 50 | 0.52 | 150 | 1.59 | 100 | 1.27 | 90 | 5 | 21 |
| C | 195 | 0.96 | 300 | 5.2 | 50 | 0.52 | 150 | 1.59 | 100 | 1.27 | 90 | 6 | 21 |
| D | 195 | 0.96 | 400 | 6.9 | 50 | 0.52 | 150 | 1.59 | 100 | 1.27 | 90 | 4 | 21 |
| E | 195 | 0.96 | 300 | 5.2 | 50 | 0.52 | 150 | 1.59 | 50 | 0.63 | 90 | 4 | 21 |
| 24 | 195 | 0.96 | 100 | 1.7 | 100 | 1.04 | 300 | 3.18. | 0 | 0 | 95 | 6 | 21 |
| 25 | 195 | 0.96 | 100 | 1.7 | 100 | 1.04 | 330 | | 0 | 0 | 90 | 6 | 21 |
| 26 | 195 | 0.96 | 100 | 1.7 | 100 | 1.04 | 360 | 3.82 | 0 | 0 | 90 | 6 | 21 |
| 27 | 195 | 0.96 | 100 | 1.7 | 100 | 1.04 | 400 | | 0 | 0 | 90 | 6 | 21 |
| * Calculated on a dried basis | | | | | | | | | | | | | |

Table 2

| (Comparative) Ex. | Molecular weight (kDA) | | 10% viscosity in acetone [mPa·s] | 2% viscosity in NaOH [mPa·s] | Methoxyl (%) | Hydroxy - propoxyl (%) | Acetyl (%) | Succi-noyl (%) | $DS_M$ | $MS_{HP}$ | $DS_{Ac}$ | $DS_s$ | Sum $DS_{Ac}$ + $DS_s$ | % water soluble at 2.5 % | Water-soluble at 2% |
|---|---|---|---|---|---|---|---|---|---|---|---|---|---|---|---|
| | Mn | Mw | | | | | | | | | | | | | |
| 1 | 25 | 89 | 12.0 | 4.3 | 26.8 | 8.5 | 7.1 | 2.1 | 1.93 | 0.26 | 0.37 | 0.05 | 0.42 | 99 | yes* |
| 2 | 25 | 114 | 13.0 | 3.8 | 26.4 | 8.3 | 7.6 | 2.1 | 1.91 | 0.25 | 0.40 | 0.05 | 0.45 | 100 | yes |
| 3 | 21 | 64 | 14.0 | 3.5 | 26.5 | 8.2 | 7.3 | 2.5 | 1.92 | 0.25 | 0.38 | 0.06 | 0.44 | 100 | yes* |
| 4 | 18 | 31 | n.m. | 3.3 | 26.4 | 8.1 | 6.8 | 3.0 | 1.91 | 0.24 | 0.35 | 0.07 | 0.42 | 100 | yes* |
| 5 | 17 | 39 | n.m. | 3.1 | 26.0 | 7.9 | 7.3 | 4.2 | 1.92 | 0.24 | 0.39 | 0.10 | 0.49 | 100 | yes |
| 6 | 18 | 29 | n.m. | 3.4 | 26.8 | 8.2 | 6.8 | 2.6 | 1.93 | 0.24 | 0.35 | 0.06 | 0.41 | 100 | yes* |
| 7 | 20 | 27 | n.m. | n.m. | 27.4 | 8.3 | 5.3 | 3.5 | 1.97 | 0.25 | 0.28 | 0.08 | 0.36 | 99 | yes* |
| 8 | 20 | 27 | n.m. | n.m. | 27.3 | 8.3 | 4.1 | 5.0 | 1.97 | 0.25 | 0.21 | 0.11 | 0.32 | 101 | yes* |
| 9 | 20 | 26 | n.m. | n.m. | 27.8 | 8.3 | 2.4 | 5.9 | 1.99 | 0.25 | 0.12 | 0.13 | 0.25 | 101 | yes* |
| 10 | 18 | 26 | n.m. | n.m. | 26.6 | 8.2 | 6.1 | 4.3 | 1.95 | 0.25 | 0.32 | 0.10 | 0.42 | 101 | yes* |
| 11 | 20 | 52 | n.m. | n.m. | 25.6 | 7.9 | 6.8 | 6.6 | 1.93 | 0.25 | 0.37 | 0.15 | 0.52 | 101 | yes |
| 12 | 22 | 57 | n.m. | n.m. | 25.1 | 7.9 | 5.3 | 8.3 | 1.90 | 0.25 | 0.29 | 0.19 | 0.48 | 100 | yes |
| 13 | 23 | 57 | n.m. | n.m. | 24.8 | 7.7 | 3.2 | 11.4 | 1.89 | 0.24 | 0.18 | 0.27 | 0.45 | 100 | yes |
| 14 | 20 | 47 | n.m. | n.m. | 25.1 | 7.9 | 6.7 | 6.6 | 1.89 | 0.25 | 0.36 | 0.15 | 0.52 | 100 | yes |
| 15 | 29 | 74 | n.m. | n.m. | 24.3 | 7.7 | 7.8 | 7.2 | 1.86 | 0.24 | 0.43 | 0.17 | 0.60 | 100 | yes |
| 16 | 27 | 67 | n.m. | n.m. | 24.2 | 7.7 | 8.0 | 7.5 | 1.86 | 0.24 | 0.44 | 0.18 | 0.62 | 96 | yes |
| 17 | 30 | 87 | n.m. | n.m. | 24.2 | 7.7 | 8.3 | 7.6 | 1.87 | 0.25 | 0.46 | 0.18 | 0.64 | 95 | yes |
| 18 | 32 | 68 | n.m. | n.m. | 24.0 | 7.8 | 7.9 | 6.7 | 1.82 | 0.24 | 0.43 | 0.16 | 0.59 | 97 | yes |
| 19 | 36 | 92 | n.m. | n.m. | 23.1 | 7.8 | 9.0 | 7.6 | 1.80 | 0.25 | 0.50 | 0.18 | 0.68 | 79 | part. |
| 20 | 58 | 176 | n.m. | n.m. | 24.2 | 7.7 | 7.3 | 8.4 | 1.87 | 0.25 | 0.41 | 0.20 | 0.61 | 81 | part. |
| 21 | 31 | 64 | n.m. | n.m. | 24.2 | 7.7 | 8.7 | 6.6 | 1.86 | 0.24 | 0.48 | 0.16 | 0.64 | 97 | yes |
| 22 | 29 | 57 | n.m. | n.m. | 23.8 | 7.7 | 9.4 | 5.9 | 1.82 | 0.24 | 0.52 | 0.14 | 0.66 | 99 | yes |
| 23 | 28 | 52 | n.m. | n.m. | 24 | 8 | 9.5 | 5.1 | 1.83 | 0.25 | 0.52 | 0.12 | 0.64 | 99 | yes |

(continued)

| (Comparative) Ex. | Molecular weight (kDA) | | 10% viscosity in acetone [mPa·s] | 2% viscosity in NaOH [mPa·s] | Methoxyl (%) | Hydroxy-propoxyl (%) | Acetyl (%) | Succi-noyl (%) | $DS_M$ | $MS_{HP}$ | $DS_{Ac}$ | $DS_s$ | Sum $DS_{Ac}$ + $DS_s$ | % water soluble at 2.5 % | Water-soluble at 2% |
|---|---|---|---|---|---|---|---|---|---|---|---|---|---|---|---|
| | Mn | Mw | | | | | | | | | | | | | |
| 23A | 17 | 33 | n.m. | n.m. | 25.4 | 8 | 8.9 | 4.8 | 1.93 | 0.25 | 0.49 | 0.11 | 0.6 | 98 | yes |
| A | 139 | 356 | 118 | n.m. | 22.0 | 6.9 | 9.2 | 14.9 | 1.88 | 0.24 | 0.57 | 0.39 | 0.96 | 10 | no |
| B | 108 | 293 | 122 | n.m. | 22.1 | 6.9 | 9.6 | 14.5 | 1.89 | 0.24 | 0.59 | 0.38 | 0.97 | 4 | no |
| c | 112 | 293 | 169 | n.m. | 21.5 | 6.8 | 9.4 | 14.3 | 1.82 | 0.24 | 0.57 | 0.37 | 0.94 | 6 | no |
| D | 29 | 80 | 15.8 | n.m. | 22.9 | 7.2 | 7.9 | 13.3 | 1.89 | 0.25 | 0.47 | 0.34 | 0.81 | 51 | no |
| E | 56 | 164 | 21.7 | n.m. | 22.5 | 7.1 | 8.4 | 13.9 | 1.88 | 0.25 | 0.51 | 0.36 | 0.86 | 30 | no |
| 24 | 22 | 65 | 23 | n.m. | 24.1 | 7.7 | 9.3 | 7.4 | 1.89 | 0.25 | 0.52 | 0.18 | 0.70 | 83 | part. |
| 25 | 23 | 60 | 122 | n.m. | 24.4 | 7.7 | 8.4 | 6.6 | 1.87 | 0.24 | 0.46 | 0.16 | 0.62 | 85 | part. |
| 26 | 27 | 73 | 479 | n.m. | 24.0 | 7.8 | 8.8 | 6.3 | 1.84 | 0.25 | 0.49 | 0.15 | 0.64 | 90 | part. |
| 27 | 22 | 64 | 89 | n.m. | 24.7 | 7.7 | 8.7 | 5.5 | 1.87 | 0.24 | 0.48 | 0.13 | 0.60 | 92 | yes |

n.m. : not measured *very clear solution part.: partially

EP 3 270 972 B1

The comparison between Examples 1 - 27 on one hand and Comparative Examples A - E on the other hand illustrates that water-soluble esters of cellulose ethers can be produced when the esterification is conducted in the presence of an aliphatic carboxylic acid but in the absence of an esterification catalyst, such as an alkali metal carboxylate. The esterified cellulose ethers of Examples 1 - 27 dissolved at a concentration of 2.0 wt.-% or more in water at a temperature of 5 °C (for the qualitative determination of the water-solubility) or at a temperature of 2 °C, respectively (for the quantitative determination of the water-solubility). In contrast thereto, the esterified cellulose ethers of Comparative Examples A - E could not brought into solution at a concentration of 2.0 wt.-% in water at a temperature of 5 °C or 2 °C. In all Comparative Examples A - E a large portion of the 2 wt.-% HPMCAS remained non-dissolved in water and formed sediment.

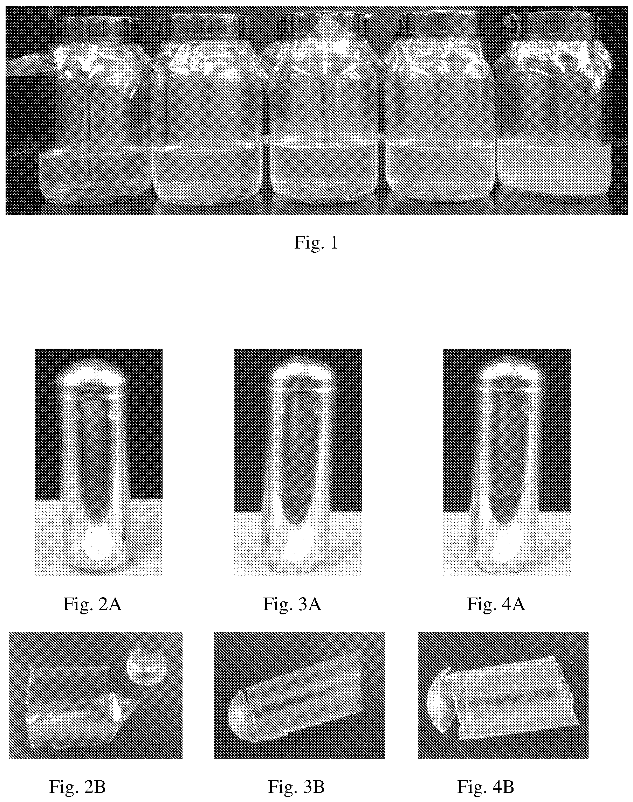

When the molar ratio [aliphatic carboxylic acid / anhydroglucose units of cellulose ether] is from [2.0 / 1] to [9.0 / 1] and in particular from [3.0 / 1] to [9.0 / 1], esterified cellulose ethers can be obtained that form very clear solutions in water. In Examples 1, 3, 4 and 6 - 10 more clear solutions were obtained than in Examples 5 and 11 at comparable molecular weights $M_w$. Fig. 1 represents a photograph of 2 wt.% solutions of the esterified cellulose ethers of Examples 7 - 11 in water.

Preparation of Capsules from Water Soluble HPMCAS of Example 15

An aqueous solution of 9.0 wt.-% of the water soluble HPMCAS of Example 15 was prepared by dissolving the HPMCAS in deionized water at a temperature of 2°C. Triethylcitrate was added as a plasticizer at an amount of 33 wt.-%, based on the weight of the HPMCAS. Capsule shells were produced by dipping metallic pins having a temperature of 21 °C, 30 °C and 55 °C, respectively, into the HPMCAS solution having a temperature of 8 °C. The pins were then withdrawn from the aqueous HPMCAS solution and a film was formed on the molding pins. Capsule shells of good quality formed on the pins at each of these temperatures. Fig. 2A, 3A and 4A are photographical representations of capsule shells on metallic pins having a temperature of 21 °C, 30 °C and 55 °C, respectively. The capsule shells formed on pins having room temperature (21 °C) were dried at room temperature, the capsule shells formed on pins having a temperature of 30 °C were dried at 30 °C and the capsule shells formed on pins having a temperature of 55 °C were dried at 55 °C. Fig. 2B, 3B and 4B are photographical representations of pieces of capsule shells formed on metallic pins having a temperature of 21 °C, 30 °C and 55 °C, respectively, after the capsule shells have been removed from the dipping pins.

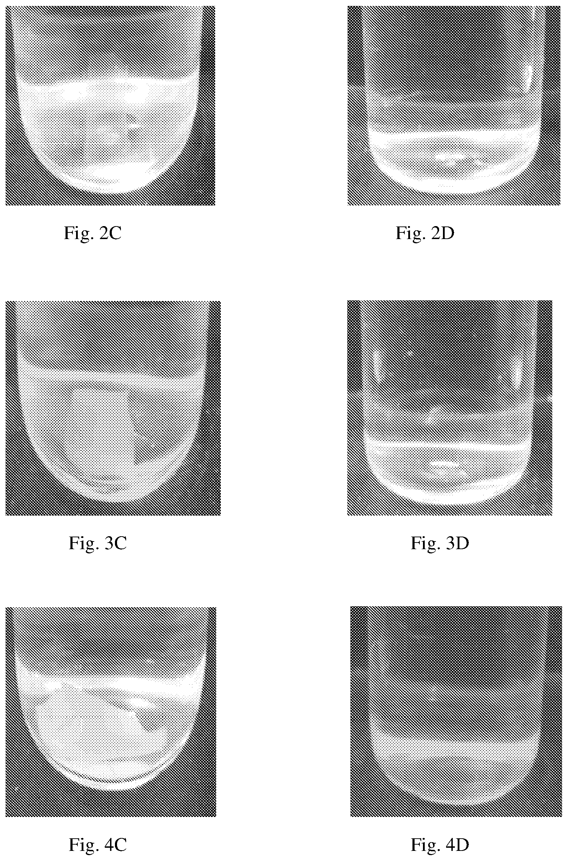

To test the solubility of the capsule shells in the acidic environment of the stomach, the capsule shells were broken into pieces and immersed into 0.1 N HCl. The capsule pieces were left there for 12 h at a temperature of 21°C. The capsule pieces did not dissolve in 0.1 N HCl during these 12 hours. The capsule pieces could be seen by the unprotected eye in 0.1 N HCl during these entire 12 hours. Fig. 2C, 3C and 4C are photographical representations of non-dissolved pieces of capsule shells in 0.1 N HCl. The pieces of capsule shells are small pieces of the capsules shells represented in Fig. 2B, 3B and 4B, respectively.

To test the solubility of the capsule shells in a neutral environment, the 0.1 N HCl was poured off from the capsule pieces and the capsule pieces were put into a McIlvaine's buffer solution (containing disodium monophosphate and citric acid) having a pH of 6.8. After about 60 minutes all pieces of capsule shells were completely dissolved in the buffer of pH 6.8 leaving clear solutions. Fig. 2D, 3D and 4D are photographical representations of the McIlvaine's buffer solution of pH 6.8 into which the non-dissolved pieces of capsule shells shown in Fig. 2C, 3C and 4C have been placed; all pieces of capsule shells are dissolved in the McIlvaine's buffer solution of pH 6.8.

Preparation of Capsules from Water Soluble HPMCAS of Example 23

An aqueous solution of 7.5 wt.-% of the water soluble HPMCAS of Example 23 was prepared by dissolving the HPMCAS in deionized water at a temperature of 2°C. Triethylcitrate was added as a plasticizer at an amount of 20 wt.-%, based on the weight of the HPMCAS. Capsule shells were produced by dipping metallic pins having a temperature of 80 °C, into the HPMCAS solution having a temperature of 10 °C. The capsule shells formed on the pins were dried at 80 °C. The prepared capsule shells had the same appearance and showed the same solubility properties in 0.1 N HCl and in aqueous buffer solution of pH 6.8 as the capsules prepared from the HPMCAS of Example 15.

**Claims**

1.  A process for preparing an ester of a cellulose ether comprising the steps of reacting a cellulose ether with a dicarboxylic acid anhydride or with a combination of a dicarboxylic acid anhydride and an aliphatic monocarboxylic acid anhydride, wherein the esterification is conducted

i) in the absence of an esterification catalyst or in the presence of no more than 0.1 mole of an esterification catalyst per mole of anhydroglucose units of cellulose ether, and
ii) in the presence of an aliphatic carboxylic acid, wherein the molar ratio [aliphatic carboxylic acid / anhydroglucose units of cellulose ether] is up to 12 / 1.

2. The process of claim 1 wherein the molar ratio [aliphatic carboxylic acid / anhydroglucose units of cellulose ether] is from [0.7 / 1] to [9.0 / 1].

3. The process of claim 2 wherein the molar ratio [aliphatic carboxylic acid / anhydroglucose units of cellulose ether] is from [3.0 / 1] to [9.0 / 1].

4. The process of any one of claims 1 to 3 wherein the esterification is conducted in the absence of an esterification catalyst or in the presence of no more than 0.02 mole of an esterification catalyst per mole of anhydroglucose units of cellulose ether.

5. The process of any one of claims 1 to 4 wherein the esterification catalyst is an alkali metal carboxylate.

6. The process of any one of claims 1 to 5 wherein the esterification is conducted in the absence of an esterification catalyst.

7. The process of any one of claims 1 to 6 wherein the cellulose ether has a viscosity of from 1.2 to 200 mPa·s, measured as a 2 weight-% solution in water at 20 °C according to ASTM D2363 - 79 (Reapproved 2006).

8. The process of claim 7 wherein the cellulose ether has a viscosity of from 2.4 to 5 mPa·s, measured as a 2 weight-% solution in water at 20 °C according to ASTM D2363 - 79 (Reapproved 2006).

9. The process of any one of claims 1 to 8 wherein the cellulose ether is an alkyl cellulose, a hydroxyalkylcellulose or a hydroxyalkyl alkylcellulose.

10. The process of claim 9 wherein the cellulose ether is hydroxypropyl methylcellulose.

11. The process of any one of claims 1 to 10 wherein the cellulose ether is reacted with a) succinic anhydride in combination with b) an aliphatic monocarboxylic acid anhydride selected from the group consisting of acetic anhydride, butyric anhydride and propionic anhydride.

12. The process of claim 11 wherein hydroxypropyl methylcellulose is reacted with succinic anhydride and acetic anhydride to produce hydroxypropyl methyl cellulose acetate succinate.

13. The process of any one of claims 1 to 12 wherein the molar ratio between the anhydride of an aliphatic monocarboxylic acid and the anhydroglucose units of the cellulose ether is from 0.1 / 1 to 7 / 1.

14. The process of any one of claims 1 to 13 wherein the molar ratio between the anhydride of a dicarboxylic acid and the anhydroglucose units of cellulose ether is from 0.1 / 1 to 3.2/1.

15. The process of any one of claims 1 to 14 wherein the esterification is conducted at a temperature of from 70 °C to 110 °C.

**Patentansprüche**

1. Verfahren zur Herstellung eines Esters eines Celluloseethers, umfassend die Schritte des Umsetzens eines Celluloseethers mit einem Dicarbonsäureanhydrid oder mit einer Kombination eines Dicarbonsäureanhydrids und eines aliphatischen Monocarbonsäureanhydrids, wobei die Veresterung

i) in Abwesenheit eines Veresterungskatalysators oder in Gegenwart von nicht mehr als 0,1 mol eines Veresterungskatalysators pro mol Anhydroglucoseeinheiten des Celluloseethers und
ii) in Gegenwart einer aliphatischen Carbonsäure, wobei das Molverhältnis [aliphatische Carbonsäure/Anhydroglucoseeinheit des Celluloseethers] bis zu 12/1 beträgt,

durchgeführt wird.

2. Verfahren nach Anspruch 1, wobei das Molverhältnis [aliphatische Carbonsäure/Anhydroglucoseeinheit des Celluloseethers] von [0,7/1] bis [9,0/1] beträgt.

3. Verfahren nach Anspruch 2, wobei das Molverhältnis [aliphatische Carbonsäure/Anhydroglucoseeinheit des Celluloseethers] von [3,0/1] bis [9,0/1] beträgt.

4. Verfahren nach einem der Ansprüche 1 bis 3, wobei die Veresterung in Abwesenheit eines Veresterungskatalysators oder in Gegenwart von nicht mehr als 0,02 mol eines Veresterungskatalysators pro mol Anhydroglucoseeinheit des Celluloseethers durchgeführt wird.

5. Verfahren nach einem der Ansprüche 1 bis 4, wobei der Veresterungskatalysator ein Alkalimetallcarboxylat ist.

6. Verfahren nach einem der Ansprüche 1 bis 5, wobei die Veresterung in Abwesenheit eines Veresterungskatalysators durchgeführt wird.

7. Verfahren nach einem der Ansprüche 1 bis 6, wobei der Celluloseether eine Viskosität von 1,2 bis 200 mPa·s, gemessen als 2-gew.-%ige Lösung in Wasser bei 20°C gemäß ASTM D2363-79 (Reapproved 2006) aufweist.

8. Verfahren nach Anspruch 7, wobei der Celluloseether eine Viskosität von 2,4 bis 5 mPa·s, gemessen als 2-gew.-%ige Lösung in Wasser bei 20°C gemäß ASTM D2363-79 (Reapproved 2006) aufweist.

9. Verfahren nach einem der Ansprüche 1 bis 8, wobei der Celluloseether eine Alkylcellulose, eine Hydroxyalkylcellulose oder eine Hydroxyalkylalkylcellulose ist.

10. Verfahren nach Anspruch 9, wobei der Celluloseether Hydroxypropylmethylcellulose ist.

11. Verfahren nach einem der Ansprüche 1 bis 10, wobei der Celluloseether mit a) Bernsteinsäureanhydrid in Kombination mit b) einem Anhydrid einer aliphatischen Monocarbonsäure, ausgewählt aus der Gruppe bestehend aus Essigsäureanhydrid, Buttersäureanhydrid und Propionsäureanhydrid, umgesetzt wird.

12. Verfahren nach Anspruch 11, wobei Hydroxypropylmethylcellulose mit Bernsteinsäureanhydrid und Essigsäureanhydrid umgesetzt wird, um Hydroxypropylmethylcelluloseacetatsuccinat herzustellen.

13. Verfahren nach einem der Ansprüche 1 bis 12, wobei das Molverhältnis zwischen dem Anhydrid einer aliphatischen Monocarbonsäure und den Anhydroglucoseeinheiten des Celluloseethers von 0,1/1 bis 7/1 beträgt.

14. Verfahren nach einem der Ansprüche 1 bis 13, wobei das Molverhältnis zwischen dem Anhydrid einer Dicarbonsäure und den Anhydroglucoseeinheiten des Celluloseethers von 0,1/1 bis 3,2/1 beträgt.

15. Verfahren nach einem der Ansprüche 1 bis 14, wobei die Veresterung bei einer Temperatur von 70°C bis 110°C durchgeführt wird.

**Revendications**

1. Un procédé pour la préparation d'un ester d'un éther de cellulose comprenant les étapes consistant à faire réagir un éther de cellulose avec un anhydride d'acide dicarboxylique ou avec une combinaison d'un anhydride d'acide dicarboxylique et d'un anhydride d'acide monocarboxylique aliphatique, dans lequel l'estérification est menée

i) en l'absence d'un catalyseur d'estérification ou en présence de pas plus de 0,1 mole d'un catalyseur d'estérification par mole d'unités anhydroglucose d'éther de cellulose, et
ii) en présence d'un acide carboxylique aliphatique, dans lequel le rapport molaire [acide carboxylique aliphatique/unités anhydroglucose d'éther de cellulose] va jusqu'à 12/1.

2. Le procédé de la revendication 1 dans lequel le rapport molaire [acide carboxylique aliphatique/unités anhydroglucose d'éther de cellulose] va de [0,7/1] à [9,0/1].

3. Le procédé de la revendication 2 dans lequel le rapport molaire [acide carboxylique aliphatique/unités anhydroglucose d'éther de cellulose] va de [3,0/1] à [9,0/1].

4. Le procédé de l'une quelconque des revendications 1 à 3 dans lequel l'estérification est menée en l'absence d'un catalyseur d'estérification ou en présence de pas plus de 0,02 mole d'un catalyseur d'estérification par mole d'unités anhydroglucose d'éther de cellulose.

5. Le procédé de l'une quelconque des revendications 1 à 4 dans lequel le catalyseur d'estérification est un carboxylate de métal alcalin.

6. Le procédé de l'une quelconque des revendications 1 à 5 dans lequel l'estérification est menée en l'absence d'un catalyseur d'estérification.

7. Le procédé de l'une quelconque des revendications 1 à 6 dans lequel l'éther de cellulose a une viscosité allant de 1,2 à 200 mPa•s, mesurée sous forme de solution à 2 % en poids dans l'eau à 20 °C conformément à ASTM D2363 - 79 (réapprouvée en 2006).

8. Le procédé de la revendication 7 dans lequel l'éther de cellulose a une viscosité allant de 2,4 à 5 mPa•s, mesurée sous forme de solution à 2 % en poids dans l'eau à 20 °C conformément à ASTM D2363 - 79 (réapprouvée en 2006).

9. Le procédé de l'une quelconque des revendications 1 à 8 dans lequel l'éther de cellulose est une alkylcellulose, une hydroxyalkylcellulose ou une hydroxyalkylalkylcellulose.

10. Le procédé de la revendication 9 dans lequel l'éther de cellulose est l'hydroxypropylméthylcellulose.

11. Le procédé de l'une quelconque des revendications 1 à 10 dans lequel l'éther de cellulose est mis à réagir avec a) de l'anhydride succinique en combinaison avec b) un anhydride d'acide monocarboxylique aliphatique sélectionné dans le groupe constitué de l'anhydride acétique, de l'anhydride butyrique et de l'anhydride propionique.

12. Le procédé de la revendication 11 dans lequel l'hydroxypropylméthylcellulose est mise à réagir avec de l'anhydride succinique et de l'anhydride acétique afin de produire de l'acétate-succinate d'hydroxypropylméthylcellulose.

13. Le procédé de l'une quelconque des revendications 1 à 12 dans lequel le rapport molaire entre l'anhydride d'un acide monocarboxylique aliphatique et les unités anhydroglucose de l'éther de cellulose va de 0,1/1 à 7/1.

14. Le procédé de l'une quelconque des revendications 1 à 13 dans lequel le rapport molaire entre l'anhydride d'un acide dicarboxylique et les unités anhydroglucose d'éther de cellulose va de 0,1/1 à 3,2/1.

15. Le procédé de l'une quelconque des revendications 1 à 14 dans lequel l'estérification est menée à une température allant de 70 °C à 110 °C.

Fig. 1

Fig. 2A

Fig. 3A

Fig. 4A

Fig. 2B

Fig. 3B

Fig. 4B

Fig. 2C

Fig. 2D

Fig. 3C

Fig. 3D

Fig. 4C

Fig. 4D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4226981 A
- EP 0219426 A
- WO 2014031447 A
- WO 2014031448 A
- WO 2014031446 A
- WO 2005115330 A
- US 4365060 A
- EP 1141029 A
- EP 210917 A
- EP 1423433 A
- US 4316982 A
- US 13030394 W
- WO 2013148154 A
- WO 2014137777 A

### Non-patent literature cited in the description

- **MCGINITY, JAMES W.** Aqueous Polymeric Coatings for Pharmaceutical Dosage Forms. M. Dekker, 1989, 105-113
- **G. BARTELMUS ; R. KETTERER.** *Z. Anal. Chem.,* 1977, vol. 286, 161-190
- Hypromellose Acetate Succinate. United States Pharmacopia and National Formulary, NF. vol. 29, 1548-1550
- Hypromellose. United States Pharmacopeia and National Formulary, USP. vol. 35, 3467-3469
- *Journal of Pharmaceutical and Biomedical Analysis,* 2011, vol. 56, 743